# DEMANDE DE BREVET EUROPEEN

(11) **EP 0 835 640 A1**
(43) Date de publication de la demande: **15.04.1998**
(21) Numéro de dépôt: 96420310.3
(22) Date de dépôt: 11.10.1996
(51) Int. Cl.: A61B 17/04, A61F 2/08

(54) **Dispositif d'ancrage d'un fil de suture dans une partie d'os notamment**

(71) Demandeur: Comte, Georges, F-21000 Dijon (FR)
(72) Inventeur: Comte, Georges, F-21000 Dijon (FR)
(74) Mandataire: Thivillier, Patrick

(57) **Abrégé**

Le dispositif d'ancrage d'un fil de suture dans une partie d'os notamment comprend un élément support (1) recevant le fil de suture (2) et un organe de manoeuvre (O) coopérant avec ledit élément. Selon l'invention, il est revendiqué que l'élément support présente :
- une partie active apte à assurer un autotaraudage et un ancrage dans l'os,
- une partie d'entrainement apte à coopérer avec une partie complémentaire que présente l'extrémité de l'organe de manoeuvre,
- une partie de fixation apte à assurer la liaison, avec capacité de déplacement du fil de suture (2) qui coopère avec des agencements de l'organe de manoeuvre (O).

## Description

L'invention se rattache au secteur des techniques chirugicales pour l'ancrage d'un fil de suture dans une substance osseuse.

De manière connue, le fil de suture est relié à un élément support destiné à être ancré dans la substance osseuse considérée. Cette technique est utilisée dans différents cas, parmi lesquels on peut citer, à titre indicatif, nullement limitatif, des réinsertions ligamentaires, des réinsertions tendinomusculaires et toutes autres indications chirurgicales spécifiques.

La mise en place et l'ancrage de l'élément support recevant le fil de suture, peut s'effectuer de différentes façons. Par exemple, le brevet EP 0 217 541 décrit un système d'ancrage dans lequel l'élément support recevant le fil de suture, a une forme générale cylindrique, dont l'une des extrémités constitue un foret pour réaliser un trou dans la substance osseuse. Cette extrémité, en forme de foret, est prolongée par une portée présentant une pluralité de saillies de filetage destinées à assurer l'ancrage dans le trou préalablement fait par le foret.

Le fil de suture est rendu solidaire d'une pièce rapportée sous forme d'un disque engagé dans un alésage de l'élément support. L'élément support coopère avec une tige d'entrainement susceptible d'être accouplée à un mandrin d'un appareil d'entrainement en rotation du type perceuse.

Cette solution nécessite, de la part de l'opérateur, une certaine dextérité, afin de bien positionner l'élément support recevant le fil de suture, à l'endroit désiré.

Cet état de la technique peut être illustré par l'enseignement du brevet US 5411506. Dans ce brevet, l'élément d'ancrage présente une partie d'ancrage en tant que telle, prolongée par une partie d'entrainement apte à coopérer avec une partie complémentaire que présente un organe de manoeuvre, cette partie d'entrainement étant prolongée par une partie de fixation percée de part en part pour le passage d'un fil de suture. La partie d'ancrage en tant que telle est de forme générale cylindrique et nécessite, pour sa mise en place dans la partie d'os considérée, un appareil d'entrainement en rotation du type perceuse. Comme indiqué précédemment, cette nécessité d'utiliser un appareil du type perceuse n'est pas très rationnelle et demande, de la part de l'opérateur, une certaine dextérité. On observe également qu'il est, dans ce cas, difficile d'obtenir une grande précision.

Par ailleurs, compte-tenu des agencement de l'accouplement du fil de suture, par rapport à l'élément support qui coopère avec une tige de diamètre réduit pour être entrainée par un mandrin, il n'est pas possible de prééquiper les extrémités du fil de suture, d'aiguilles qui sont indispensables à l'intervention chirurgicale considérée. Il est donc nécessaire, dans ce cas, de fixer les aiguilles aux extrémités du fil de suture, après l'ancrage de l'élément support. On conçoit qu'une telle méthode n'est pas rationnelle.

On connaît également d'autres solutions dans lesquelles l'élément support est constitué par un élément de forme générale cylindrique présentant en débordement de ses génératrices, des languettes flexibles d'ancrage. L'extrémité de l'élément est biseautée pour faciliter son introduction dans un trou qu'il est nécessaire de pratiquer préalablement dans la partie d'os considérée. Après engagement de l'élément support dans le trou, les ailettes ont tendance à s'écarter pour assurer l'ancrage. La mise en place de l'élément support s'effectue par impaction au moyen d'un outil approprié. A noter que dans ce cas, l'élément support recoit le fil de suture qui peut préalablement, être équipé des aiguilles.

Par contre, avec cette solution, après impaction de l'élément support, ce dernier est indémontable, ce qui peut poser des problèmes dans certains cas.

L'invention s'est fixée pour but de remédier à ces inconvénients, de manière simple, sûre, efficace et rationnelle.

Le problème que se propose de résoudre l'invention est de faciliter et simplifier l'ancrage de l'élément support dans la substance osseuse, tout en pouvant équiper d'aiguilles les extrémités du fil de suture, préalablement à l'ancrage de l'élément support, en ayant, par ailleurs, la possibilité de facilement démonter l'élément support si nécessaire.

Plus particulièrement, pour résoudre le problème posé d'assurer facilement l'ancrage de l'élément support dans la substance osseuse, sans nécessiter l'emploi d'appareils particuliers du type perceuse, une partie active de l'élément d'ancrage présente en combinaison :
- une zone d'extrémité pointue faisant office de pointeau,
- une zone tronconique présentant un filetage pour assurer une fonction d'auto-taraudage,
- une zone cylindrique où se termine ledit filetage pour assurer l'ancrage dans la partie osseuse.

Compte-tenu de ces dispositions, il suffit donc, après avoir déterminé l'endroit précis de la substance osseuse où doit être ancré l'élément d'exercer une force d'appui pour assurer l'impaction de la zone d'extrémité qui fait office de pointeau, puis de visser manuellement l'élément à la façon d'une vrille.

Pour résoudre le problème posé d'assurer l'entrainement de l'élément support, la partie d'entrainement fait suite coaxialement à la zone cylindrique de la partie active et est constituée par une portée mâle polygonale coopérant avec une portée femelle complémentaire formée en bout d'une tige que présente l'organe de manoeuvre.

Pour résoudre le problème posé d'équiper l'élément support du fil de suture, la partie de fixation du fil de suture est disposée coaxialement à la partie d'entrainement et présente un trou pour le libre passage du fil de suture en vue de son engagement dans les agencements de l'organe de manoeuvre.

Les agencements sont constitués par au moins une fente formée en bout de la tige et selon ses génératrices, pour l'engagement du fil de suture.

Pour résoudre le problème posé de pouvoir déterminer le moment où l'élément support est correctement ancré dans la substance osseuse, la portée femelle délimite, avec l'extrémité de la tige, un épaulement faisant office de butée avec l'os en position d'ancrage de l'élément support.

Pour résoudre le problème posé d'assurer la manoeuvre de l'élément support, tout en ayant pour objectif d'incorporer le fil de suture, la tige est solidaire d'un manche de préhension qui présente des moyens pour le positionnement et le blocage temporaire du fil de suture.

Les moyens sont constitués par un évidement dans lequel est logé le fil de suture, ledit évidement présentant au moins un pion débordant pour enrouler partiellement le fil de suture en vue de son blocage temporaire en combinaison avec des fentes et pour assurer, de manière concomitante, l'accouplement de l'élément support en bout de la tige de l'organe de manoeuvre.

Pour résoudre le problème posé d'assurer le maintien du fil de suture par rapport à l'organe de manoeuvre, l'évidement du manche est obturé par une plaque amovible en matériau souple conformée pour exercer un effort de pression sur le fil de suture.

Suivant une autre caractéristique, les extrémités du fil de suture sont équipées d'aiguilles serties positionnées dans l'évidement du manche.

Pour permettre à l'opérateur de percevoir d'une manière tactile, la mise en place de l'élément support, la tige déborde de l'extrémité libre du manche.

L'invention est exposée, ci-après plus en détail à l'aide des dessins annexés, dans lesquels :
La figure 1 est une vue en perspective de l'ensemble du dispositif.

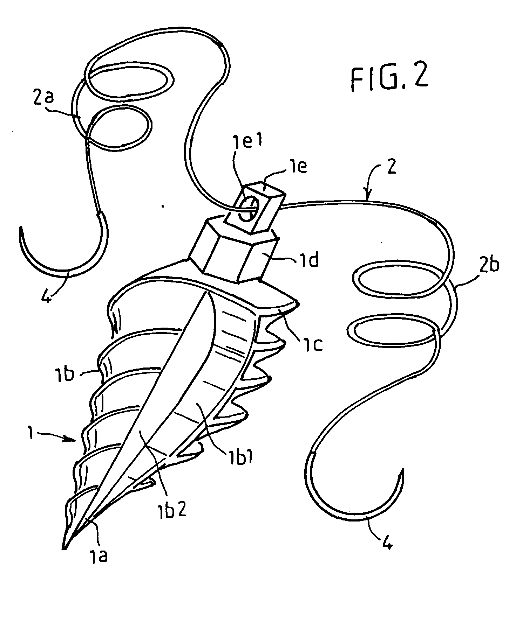

La figure 2 est une vue en perspective de l'élément support équipé du fil de suture.

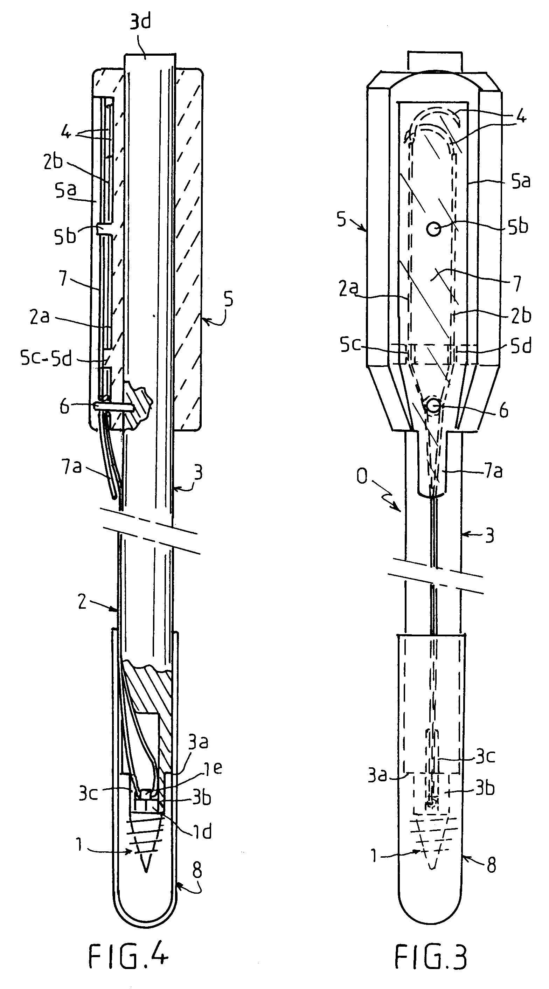

La figure 3 est une vue de face de l'ensemble du dispositif.
La figure 4 est une vue en coupe longitudinale correspondant à la figure 3.

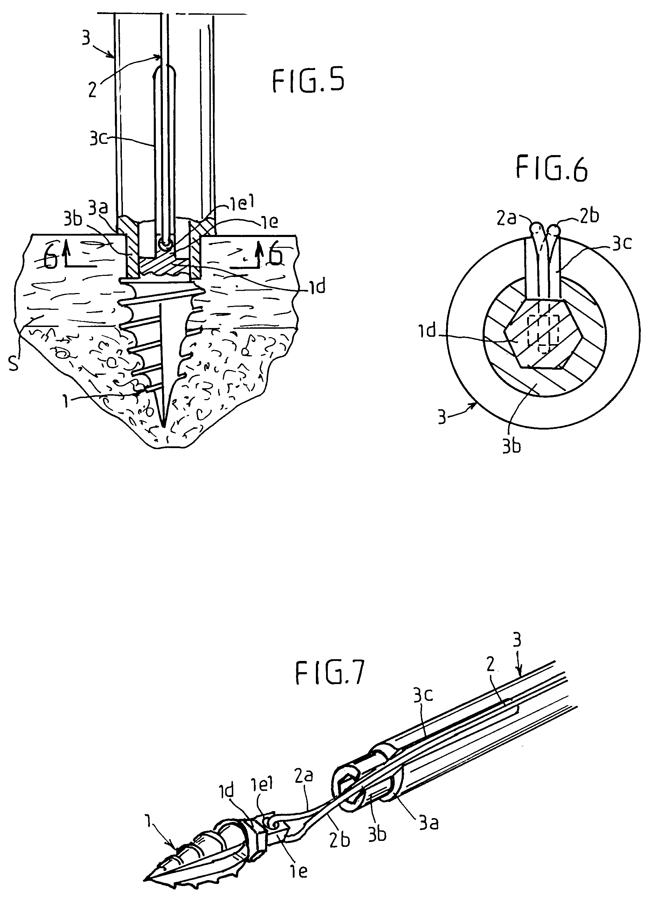

La figure 5 est une vue partielle en coupe longitudinale montrant l'ancrage de l'élément support du fil de suture.
La figure 6 est une vue en coupe transversale considérée selon la ligne 6.6 de la figure 4.
La figure 7 est une vue partielle en perspective avant accouplement et mise en tension de l'élément d'ancrage en bout de la tige.

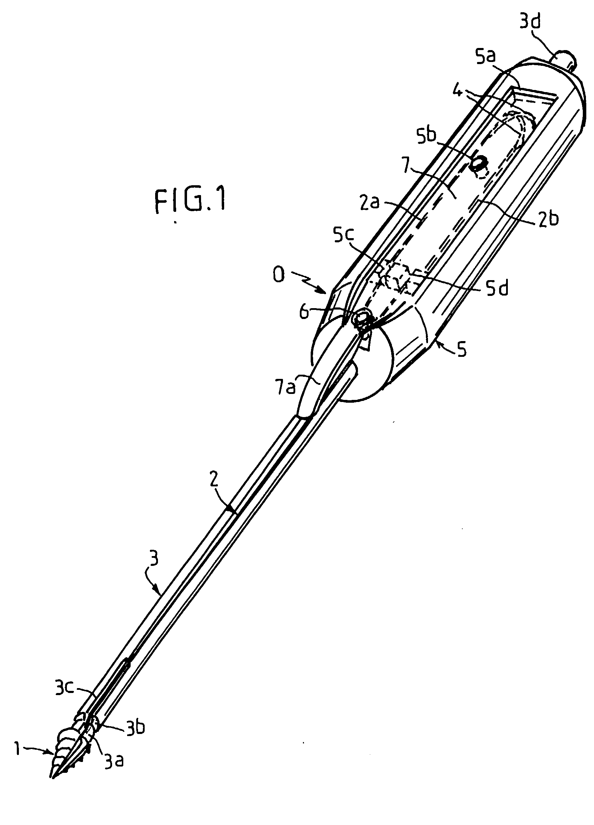

Comme le montre la figure 1, le dispositif d'ancrage comprend essentiellement un élément support (1) recevant le fil de suture (2) et un organe de manoeuvre (O) coopérant avec ledit élément.

Selon une caractéristique importante de l'invention, l'élément support (1) comprend une partie active apte à assurer un autotaraudage et un ancrage dans la substance osseuse considérée (S). Plus particulièrement, la partie active de l'élément support (1) présente une zone d'extrémité pointue (1a) faisant office de pointeau. Cette zone d'extrémité (1a) est prolongée coaxialement par une portée tronconique (1b) présentant un filetage (1b1), pour assurer la fonction d'autotaraudage. Le filetage (1b1) se termine par une portée cylindrique (1c), pour assurer l'ancrage dans la partie osseuse. La portée cylindrique (1c) est de hauteur réduite par rapport à la portée tronconique (1b).

A noter que la portée tronconique (1b) d'autotaraudage peut présenter selon ses génératrices, une gorge (1b2) pour le dégagement des copeaux résultant de l'autotaraudage de la substance osseuse.

La partie active est prolongée coaxialement du côté de la portée cylindrique (1c) par une portée polygonale (1d) apte à assurer l'entrainement de l'élément (1) au moyen de l'organe de manoeuvre (O). Cette portée polygonale (1d) coopère avec une portée femelle complémentaire (3a) formée en bout d'une tige (3), que présente l'organe de manoeuvre. Cette portée femelle (3a) délimite avec l'extrémité de la tige (3), un épaulement (3b). Comme il sera indiqué dans la suite de la description, cet épaulement (3b) fait office de butée avec l'os, en position d'ancrage de l'élément support (1).

La portée polygonale d'entrainement (1d) est elle-même prolongée coaxialement par une partie (1e) percée de part en part en (1e1) pour le libre passage du fil de suture (2) équipé, à chacune de ses extrémités, d'aiguilles (4). De manière connue, les aiguilles (4) sont serties en bout du fil de suture (2).

Le fil (2) délimite de part et d'autre de la partie (1e) deux brins (2a) et (2b) qui sont engagés dans une fente (3c) que présente la portée femelle d'entrainement (3a) et l'extrémité de la tige (3). Cette fente (3c) est formée selon les génératrices de la tige (3). Les deux brins (2a) et (2b), engagés dans la fente (3c), suivent les génératrices de la tige (3), pour coopérer avec des agencements et moyens de blocage temporaires que présente un manche (5) rendu solidaire de l'extrémité supérieure de la tige (3).

Le manche (5) présente, très sensiblement dans sa partie médiane, un évidement (5a), pour le logement de l'ensemble du fil de suture (2), y compris les aiguilles (4), pour assurer la mise en tension du fil de suture (2) et, d'une manière concomitante, l'accouplement de l'élément (1) en bout de la tige (3). L'évidement (5a) débouche du côté de la partie de raccordement entre le manche (5) et la tige (3), et présente au moins un pion débordant (6). Ce pion permet d'enrouler successivement chacun des brins (2a) et (2b) du fil de suture, en vue de sa mise en tension. Les brins sont ensuite engagés séparément dans deux fentes (5c) que présente un épaulement (5d) formé transversalement dans l'évidement (5a).

A noter que le pion (6) peut être formé en débordement de la tige (3) pour améliorer sa solidarisation avec le manche (5).

L'évidement (5a) du manche (5) est obturé par une plaque amovible (7) en matériau souple et transparent notamment. Cette plaque (7) présente un nez profilé (7a) apte à prendre appui sur les brins (2a) et (2b) du fil de suture (2), en vue de son positionnement par rapport à la tige (3). La plaque (1) a une forme complémentaire de celle de l'évidement (5a) en y étant par exemple maintenue par un pion (5b).

Enfin, il apparait que l'extrémité de la tige (3) déborde en (3d) du manche (5) pour permettre à l'opérateur de mieux ressentir l'ancrage de l'élément (1).

L'utilisation du dispositif selon l'invention, est particulièrement simple et efficace.

L'élément support (1) est monté en bout de la tige (3) en y étant maintenu en tension par le fil de suture (2). Les brins (2a) et (2b) du fil sont engagés dans la fente (3c) pour être logés dans l'évidement (5a) du manche (5), en étant équipés des aiguilles (4). La plaque (7) permet d'obturer temporairement l'évidement (5a), tout en permettant le positionnement du fil (2) par rapport à la tige (3).

Pour assurer l'ancrage de l'élément (1), l'opérateur, après avoir déterminé l'endroit précis de la substance osseuse où doit être ancré ledit élément, exerce en bout de la tige (3d), une force d'appui pour assurer l'impaction de la zone d'extrémité (la) faisant office de pointeau. Il suffit ensuite de visser l'élément (1) au moyen du manche (5), pour provoquer l'enfoncement dudit élément (1) par son profil autotaraudant (1b1) et son ancrage par sa partie (1c). Le vissage s'effectue jusqu'en position de butée de l'épaulement (3b) de la tige (3) sur la substance osseuse.

Il suffit ensuite de retirer l'ensemble de l'organe de manoeuvre, après avoir libéré le fil de suture (2) du manche (5). L'élément (1) est alors ancré dans la substance osseuse, en étant équipé du fil de suture (2) dont les extrémités reçoivent les aiguilles (4).

Les avantages ressortent bien de la description, en particulier on souligne et'on rappelle :
- la suppression de tout organe rapporté du type perceuse, afin de s'affranchir du problème de la stérilisation,
- la possibilité d'équiper préalablement les extrémités du fil de suture des aiguilles,
- la facilité de manipulation par l'opérateur,
- le coût de revient réduit,
- l'ergonomie de l'ensemble du dispositif,
- la possibilité de démonter l'élément d'ancrage.

Bien évidemment, l'extrémité de la tige (3) équipée de l'élément d'ancrage (1), reçoit un embout protecteur (8).

## Revendications

1. Dispositif d'ancrage d'un fil de suture dans une partie d'os notamment comprenant un élément support (1) recevant le fil de suture (2) et un organe de manoeuvre (O) coopérant avec ledit élément qui présente :
- une partie active (1a) (1b) (1c) d'ancrage dans l'os,
- une partie d'entrainement (1d) apte à coopérer avec une partie complémentaire que présente l'extrémité de l'organe de manoeuvre,
- une partie de fixation (1e) apte à assurer la liaison, avec capacité de déplacement, du fil de suture (2) qui coopère avec des agencements de l'organe de manoeuvre (O)
caractérisé en ce que la partie active de l'élément support (1) présente successivement :
- une zone d'extrémité pointue (1a) faisant office de pointeau,
- une zone tronconique (1b) présentant un filetage (1b1) pour assurer une fonction d'auto-taraudage,
- une zone cylindrique (1c) où se termine ledit filetage (1b1) pour assurer l'ancrage dans la partie osseuse.

2. Dispositif selon la revendication 1, caractérisé en ce que la partie d'entrainement (1d) fait suite coaxialement à la zone cylindrique (1c) de la partie active et est constituée par une portée mâle polygonale coopérant avec une portée femelle complémentaire (3a) formée en bout d'une tige (3) que présente l'organe de manoeuvre (O).

3. Dispositif selon la revendication 1, caractérisé en ce que la partie de fixation (1e) du fil de suture (2) est disposée coaxialement à la partie d'entrainement (1d) et présente un trou (1e1) pour le libre passage du fil de suture en vue de son engagement dans les agencements de l'organe de manoeuvre (O).

4. Dispositif selon la revendication 3, caractérisé en ce que les agencements sont constitués par au moins une fente (3c) formée en bout de la tige (3) et selon ses génératrices, pour l'engagement du fil de suture (2).

5. Dispositif selon les revendications 2 et 4, caractérisé en ce que la portée femelle (3a) délimite, avec l'extrémité de la tige, un épaulement (3b) faisant office de butée avec l'os en position d'ancrage de l'élément support (1).

6. Dispositif selon la revendication 2, caractérisé en ce que la tige (3) est solidaire d'un manche de préhension (5) qui présente des moyens (5a) (6) pour le positionnement et le blocage temporaire du fil de suture (2).

7. Dispositif selon la revendication 6, caractérisé en ce que les moyens sont constitués par un évidement (5a) dans lequel est logé le fil de suture (2), ledit évidement (5a) présentant au moins un pion débordant (6) pour enrouler partiellement le fil de suture en vue de son blocage temporaire en combinaison avec des fentes (5c) et pour assurer, de manière concomitante, l'accouplement de l'élément support (1) en bout de la tige de l'organe de manoeuvre (O).

8. Dispositif selon la revendication 7, caractérisé en ce que l'évidement (5a) du manche (5) est obturé par une plaque amovible (7) en matériau souple conformée pour exercer un effort de pression sur le fil de suture (2).

9. Dispositif selon la revendication 1, caractérisé en ce que les extrémités du fil de suture (2) sont équipées d'aiguilles (4), serties positionnées dans l'évidement du manche.

10. Dispositif selon la revendication 2, caractérisé en ce que la tige (3) déborde en (3d) de l'extrémité libre du manche.
